# EUROPEAN PATENT SPECIFICATION

(11) **EP 4 198 126 B1**
(45) Date of publication and mention of the grant of the patent: **15.04.2026**
(21) Application number: 21215114.6
(22) Date of filing: 16.12.2021
(51) Int. Cl.: C12N 15/10

(54) **COMPOSITION COMPRISING A DNA-DEPENDENT PROTEIN KINASE CATALYTIC SUBUNIT INHIBITOR AND 53BP1 INHIBITOR, GENE EDITING METHOD, ENGINEERED CELLS, AND USE OF THE COMPOSITION IN GENE EDITING**
ZUSAMMENSETZUNG MIT EINEM DNA-ABHÄNGIGEN PROTEINKINASEKATALYSATORUNTEREINHEITINHIBITOR UND EINEM 53BP1-INHIBITOR, GENEDITIERVERFAHREN, GENTECHNISCH MANIPULIERTE ZELLEN UND VERWENDUNG DER ZUSAMMENSETZUNG IM GEN-EDITING
COMPOSITION COMPRENANT UN INHIBITEUR DE SOUS-UNITÉ CATALYTIQUE DE PROTÉINE KINASE ADN-DÉPENDANTE ET UN INHIBITEUR DE 53BP1, PROCÉDÉ D'ÉDITION DE GÈNES, CELLULES MODIFIÉES ET UTILISATION DE LA COMPOSITION DANS L'ÉDITION DE GÈNES

(43) Date of publication of application: 21.06.2023
(73) Proprietor: Schumann, Kathrin, 81545 München (DE); Westmeyer, Gil Gregor, 80333 München (DE); Truong, Dong-Jiunn Jeffery, 85356 Freising (DE); Ursch, Laurenz, 80799 München (DE)
(72) Inventor: Schumann, Kathrin, 81545 München (DE); Westmeyer, Gil Gregor, 80333 München (DE); Truong, Dong-Jiunn Jeffery, 85356 Freising (DE); Ursch, Laurenz, 80799 München (DE)
(74) Representative: Grund, Martin

(56) References cited:
- WO-A2-2021/232014
- CN-A- 110 951 784
- US-A1- 2021 008 161
- US-B2- 10 808 017
- DANIEL KRANSER ET AL: "Increasing HR-Mediated Genome Editing in HSPCs through Manipulation of DNA Repair Proteins", MOLECULAR THERAPY, vol. 26, no. 5(suppl 1), 1 May 2018 (2018-05-01), GB, pages 84, XP055591295, ISSN: 1525-0024, DOI: https://doi.org/10.1016/j.ymthe.2018.05.001
- MARELLA D CANNY ET AL: "Inhibition of 53BP1 favors homology-dependent DNA repair and increases CRISPR?Cas9 genome-editing efficiency", NATURE BIOTECHNOLOGY, vol. 36, no. 1, 27 November 2017 (2017-11-27), New York, pages 95 - 102, XP055487471, ISSN: 1087-0156, DOI: 10.1038/nbt.4021
- ANASTASIA LOMOVA ET AL: "Improving Gene Editing Outcomes in Human Hematopoietic Stem and Progenitor Cells by Temporal Control of DNA Repair : Improving Gene Editing Outcomes in Human HSPCs", STEM CELLS, vol. 37, no. 2, 27 November 2018 (2018-11-27), pages 284 - 294, XP055732479, ISSN: 1066-5099, DOI: 10.1002/stem.2935
- L. YANG ET AL: "Optimization of scarless human stem cell genome editing", NUCLEIC ACIDS RESEARCH, vol. 41, no. 19, 31 July 2013 (2013-07-31), pages 9049 - 9061, XP055113989, ISSN: 0305-1048, DOI: 10.1093/nar/gkt555
- ANONYMOUS: "GSN:BHR36413, pcCAG-PBD2 vector DNA, SEQ ID:1.", 11 June 2020 (2020-06-11), XP055924410, Retrieved from the Internet <URL:http://ibis.internal.epo.org/exam/dbfetch.jsp?id=GSN:BHR36413> [retrieved on 20220524]
- UJJAYINEE RAY ET AL: "Modulation of DNA double-strand break repair as a strategy to improve precise genome editing", ONCOGENE, 3 September 2020 (2020-09-03), London, XP055728209, ISSN: 0950-9232, DOI: 10.1038/s41388-020-01445-2
- DANNER ERIC ET AL: "Control of gene editing by manipulation of DNA repair mechanisms", MAMMALIAN GENOME, SPRINGER NEW YORK LLC, US, vol. 28, no. 7, 3 April 2017 (2017-04-03), pages 262 - 274, XP036304552, ISSN: 0938-8990, [retrieved on 20170403], DOI: 10.1007/S00335-017-9688-5
- CHEN SIWEI ET AL: "Modulating CRISPR/Cas9 genome-editing activity by small molecules", DRUG DISCOVERY TODAY, ELSEVIER, AMSTERDAM, NL, vol. 27, no. 4, 22 November 2021 (2021-11-22), pages 951 - 966, XP087000332, ISSN: 1359-6446, [retrieved on 20211122], DOI: 10.1016/J.DRUDIS.2021.11.018
- JAYME SALSMAN ET AL: "CRISPR/Cas9 Gene Editing: From Basic Mechanisms to Improved Strategies for Enhanced Genome Engineering In Vivo", CURRENT GENE THERAPY, vol. 17, no. 4, 4 January 2018 (2018-01-04), NL, XP055732543, ISSN: 1566-5232, DOI: 10.2174/1566523217666171122094629

## Description

### FIELD OF THE INVENTION

The present invention relates to a composition comprising a DNA-dependent protein kinase catalytic subunit inhibitor and a 53BP1 inhibitor. The present invention also relates to a composition comprising a DNA-dependent protein kinase catalytic subunit inhibitor and a 53BP1 inhibitor for use in medicine. Furthermore, the present invention relates to a gene editing enhancing composition comprising a DNA-dependent protein kinase catalytic subunit inhibitor and a 53BP1 inhibitor. Besides, the present invention relates to a gene editing method comprising the sequential steps of: (i) enriching isolated cells, followed by activating said isolated cells by pre-determined surface antigens; (ii) adding a composition according to the invention to said cells simultaneously to transfecting said cells two days after step (i); (iii) adding a stimulant to said cells five days after step (i); (iv) analyzing the genome editing efficiency and viability of said cells by fluorescent-activated cell sorting. Disclosed herein, but not part of the invention, is also an engineered cell obtained by the gene editing method of the present inventionand the use of the composition comprising a DNA-dependent protein kinase catalytic subunit inhibitor and a 53BP1 inhibitor in gene editing.

### INTRODUCTION

Cell therapies usually involve harvesting cells that are both rare and challenging to harvest from subjects. Additionally, genetic manipulations of the harvested cells are often required, which cause excessive cell death of up to 80%, depending on the specific cell population. Said genetic manipulations in turn frequently result in transduction rates as low as three percent (depending on the specific cell population) and are, therefore, not efficient. Moreover, it would be desirable to increase the amount of nucleic acid used in these genetic manipulations to improve further said genetic manipulations of the cells, which is another limiting factor of cell therapies. Additionally, genetic manipulations of cells often cause excessive cell death.

It is assumed that excessive cell death in genetically manipulated cells is caused by toxicity from numerous non-homologous end joining (NHEJ) events. Suppose the genetic manipulation method of choice, such as, for example, a gene editing method such as CRISPR-Cas, causes DNA double-strand breaks. In that case, these DNA double-strand breaks are repaired by two competing pathways: non-homologous end joining (NHEJ) and homology-directed repair (HDR).

There are at least two NHEJ pathways, the canonical NHEJ (cNHEJ) pathway, and the alternative NHEJ (altNHEJ) pathway. In cNHEJ, the Ku70-Ku80 heterodimer recognizes the DNA double-strand break, binds to the DNA ends, recruiting DNA-PKcs to phosphorylate Artemis, which in turn processes single-stranded overhangs, followed by ligation of the DNA ends by DNA ligase 4 and the scaffold protein XRCC4. The altNHEJ pathway is activated if the cNHEJ pathway is repressed, where altNHEJ is the more error-prone NHEJ pathway. altNHEJ involves DNA end recognition and resection by the Mrn complex, thereby exposing microhomology regions within the single strand regions, followed by bridging and aligning the DNA ends and subsequent digestion of the non-homologous 3' tails generating gaps, which are filled by polymerase (PolQ) synthesis. Then, the DNA ends are ligated by DNA ligase 3, and the scaffold protein XRCC3 binds, leading to insertion-deletions ("indels").

In contrast, HDR comprises homologous recombination (HR), which is the commonest form of HDR. Briefly, HR involves the generation of long 3' single-stranded DNA (ssDNA) overhangs, facilitated by the Mrn complex inter alia, followed by the stabilization of said overhangs. Then, strand invasion with a homologous donor DNA sequence takes place, followed by DNA synthesis, thereby resolving the Holiday junction and repairing the double-strand break. Therefore, the aim of the invention is to decrease the number of NHEJ events while increasing the number of HDR events.

There are some studies reporting on compositions comprising both a DNA-dependent protein kinase catalytic subunit inhibitor and a 53BP1 inhibitor, see for instance US 2021/008161 A1; Kranser D. et al. (2018), Molecular Therapy, vol. 26(5)(Suppl. 1):p84; Canny M. et al, (2017), Nature Biotechnology, vol. 36(1):95-102; and US 10,808,017 B2. The inventors surprisingly found that a combination comprising a DNA protein kinase catalytic subunit (DNA-Pkcs) inhibitor and a 53BP1 inhibitor increases genome editing efficiency in a highly significant manner while exhibiting low toxicity, thereby improving cell viability. In particular, the inventors surprisingly found that the 53BP1 inhibitor's main effect is the increase of cell survival instead of the reported increase (Canny et al., 2018) in HDR-mediated relative knock in efficiency. This effect is most likely due the dual role of 53BP1, which reportedly regulates NHEJ, but also positively regulates p53 activity. Thus, inhibition of 53BP1 also abrogated the general DNA damage response pathway, where activation of p53 (encoded by TP53) by 53BP1 would lead to apoptosis. The effect of an increase in relative knock in efficiency was mediated by a DNA-Pkcs inhibitor (AZD-7648), but in contrast to the 53BP1 inhibitor (named i53, i53BP1 or i53BP in the following), the DNA-Pkcs inhibitor (AZD-7648) alone did not alleviate the genome-editing mediated reduction in cell viability, and thus, the total amount of HDR-repaired cells did not increase. Therefore, the increase in absolute HDR-mediated knock-in events was facilitated by (i) the increase of the relative knock-in efficiency by a DNA-Pkcs inhibitor (AZD-7648) and by (ii) the increase of cell viability after genome editing by inhibition of 53BP1 using i53BP1. In other words, only the combination of a DNA-Pkcs inhibitor, such as for example AZD-7648 and i53BP1 leads to an increase of absolute HDR-mediated knock-in events, whereas a DNA-Pkcs inhibitor solely increases relative knock-in efficiency and i53BP1 solely increases cell viability.

Therefore, the inventive combination comprising at least one DNA-Pkcs inhibitor and 53BP1 inhibitor allows the targeted insertion of cassettes in a highly efficient manner. The inventors report a 17.5-fold increase in HDR compared to control, indicating the described targeted insertion. Simultaneously, toxicity rates are low, and the cell viability data are acceptable. Furthermore, the composition as a gene editing enhancing composition is described as well as a composition for use in medicine. Also, a gene editing method, engineered cells obtained by said method, and the use of the composition are described.

### BRIEF SUMMARY OF THE INVENTION

In one aspect, a composition comprising a DNA-dependent protein kinase catalytic subunit inhibitor and a 53BP1 inhibitor is claimed, wherein the DNA-dependent protein kinase catalytic subunit inhibitor is AZD-7648 and/or M3814 and wherein the 53BP1 inhibitor is set forth in SEQ ID NO: 17. In one embodiment, the composition comprises donor ssDNA or donor dsDNA. In one embodiment, the composition is for use in medicine. In yet another embodiment, the composition is a gene editing enhancing composition.

In another aspect, the invention relates to a gene editing method comprising the sequential steps of: (i) enriching isolated cells, followed by activating said isolated cells by pre-determined surface antigens; (ii) adding a composition according to the previous aspect and its embodiments to said cells simultaneously to transfecting said cells by electroporation or transfection reagents two days after step (i); (iii) adding a stimulant to said cells five days after step (i); (iv) analyzing the genome editing efficiency and viability of said cells by fluorescent-activated cell sorting. In one embodiment, the isolated cells of step (i) are eukaryotic cells. In another embodiment, the isolated cells of step (i) are human stem cells, preferably human hematopoietic stem cells. In yet another embodiment, a site-specific insertion of a cassette comprising between 1 and 10,000 nucleotides, preferably between 2000 and 10,000 nucleotides, into a pre-defined locus takes place in steps (ii) and (iii). In even another embodiment, the genome editing efficiency of step (iv) is improved by at least 10-fold compared to genome editing methods in which no composition as described in the previous aspect and its embodiments is added to the cells.

### DEFINITIONS

*"Gene editing enhancing composition"* refers to a composition that enhances or improves gene editing. Gene editing, according to the present invention, refers to the targeted insertion of a segment of nucleotides into target molecules, such as the targeted insertion of DNA into DNA. Therefore, the gene editing enhancing composition according to the present invention refers to a composition that improves the targeted insertion upon CRISPR-Cas gene editing, meganuclease gene editing, TALEN gene editing, or gene editing mediated by zinc finger nucleases. In particular, the gene editing enhancing composition improves gene editing editing by increasing the rate of HDR relative to NHEJ. The gene editing enhancing composition comprises at least one DNA-Pkcs inhibitor and a 53BP1 inhibitor. In some embodiments, the gene editing enhancing composition additionally comprises donor dsDNA or donor ssDNA, preferably donor ssDNA. The gene editing enhancing composition can additionally comprise, for example, cofactors or salts.

*"Targeted insertion"* refers to a process of altering a specific sequence, which can be a gene or a part of a gene at its location in any genome. According to the present invention, said alteration of a specific sequence is performed by insertion. The insertion is a targeted insertion due to highly specific gene editing tools, such as for example CRISPR-Cas gene editing, meganuclease gene editing, TALEN gene editing, or gene editing mediated by zinc finger nucleases.

*"**Donor nucleic acid molecule"*** refers to any type of nucleic acid molecule, such as RNA or DNA, either single or double-stranded.

*"Transfection"* refers to the introduction of one or more exogenous polynucleotides into a host cell by using physical or chemical methods. Alternatively, the terms "transformation" or "transduction" are used interchangeably for "transfection" herein.

*"Autologous"* refers to any material derived from the same individual to whom it is later to be re-introduced into the same individual.

***"Allogeneic"*** refers to any material derived from a different animal of the same species as the individual to whom the material is introduced. Two or more individuals are said to be allogeneic to one another when the genes at one or more loci are not identical.

***"In vitro"*** refers to studies and experiments, which are performed with, e.g., cells or tissues and in some cases, compositions such as the compositions according to the present invention outside their normal biological context, i.e., a living organism in the context of the present invention. Therefore, *in vitro* experiments in accordance with the present invention are performed, for example, in a test tube or a culture dish.

***"Ex vivo"*** refers to studies and experiments, which are performed with, e.g., cells or tissues and in some cases, biological molecules such as the compositions according to the present invention outside their normal biological context, i.e., a living organism in the context of the present invention while using the cells of a living organism. Therefore, *ex vivo* experiments in accordance with the present invention are performed, for example, in a test tube or a culture dish using cells of a living organism.

*"Inhibitor"* refers to a molecule or compound that tends to nullify the action of another, or in some instances that blocks the ability of a given chemical or interaction or binding partner, thereby preventing a biological response according to the present invention. Inhibitors are not limited per se to a specific type of compound and may include peptides, proteins, organic or inorganic compounds (for example, small molecules), or nucleotide sequences such as plasmids. The term "inhibitor" is generally synonymous with "antagonist."

***"Platinum-based compound"*** refers to any compound comprising platinum and is suitable for the treatment of diseases such as cancer.

### FIGURES

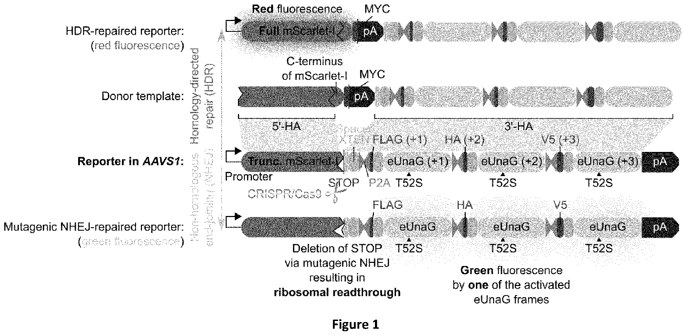

**Figure 1** shows the principle of an "enhanced traffic light reporter" (eTLR) system reporting both mutagenic end-joining events (green fluorescence), such as non-homologous end-joining (NHEJ) or microhomology-mediated end-joining (MMEJ), and homology-directed repair (HDR) events (red fluorescence) after the introduction of a double-strand break (DSB), e.g., by CRISPR/Cas9. Briefly, the system comprises a C-terminally truncated mScarlet-I red fluorescent protein followed by a stop codon and and a concatenation of three downstream P2A: eUnaG coding sequences, each in one of three possible respective translational frames. Also, all out-of-frame stop codons that lead to a premature translational termination were removed via silent mutagenesis to activate one of the eUnaG frames after a mutagenic end-joining event. The mutation T52S was introduced into eUnaG to remove an out-of-frame stop codon that could not be removed silently. Moreover, all out-of-frame ATGs were also removed, which can lead to a cryptic translational initiation and thus an undesired cryptic activation of one of the eUnaG frames. Thus, if any DNA-nick or double-strand break (DSB) at the STOP codon of the C-terminally truncated mScarlet-I is erroneously repaired, e.g., via NHEJ/MMEJ/SSA, the resulting deletion of the STOP codon by InDels leads to the subsequent ribosomal readthrough into one of the eUnaG reading frames, resulting in green fluorescence. If instead, a successful edit occurs via HDR using the donor template containing a promoterless ATG-less full coding frame of mScarlet-I and a polyadenylation site with 5 and 3' homology arms (HA), HDR-repair leads to the repair of the C-terminally truncated mScarlet-I and, therefore, yield red fluorescent cells. If desired, immunofluorescence can decode the exact frame by staining one of the three epitope tags (FLAG, HA, V5). A signal on both the red and green channels (represented by orange) indicates that the desired editing event occurred only on one allele, while the other underwent mutagenic end-joining.

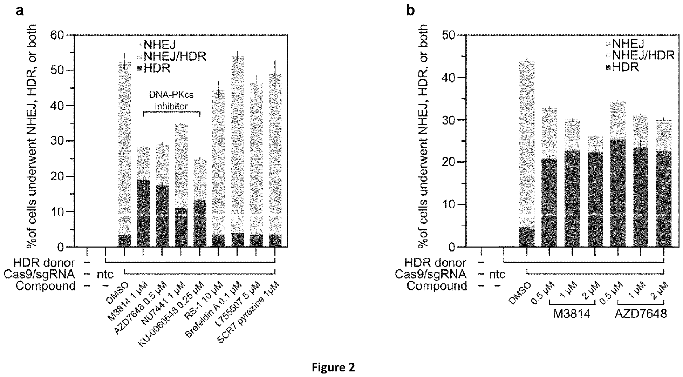

**Figure 2A** shows the FACS results 72 h after transfection of HEK293 cells carrying two stably integrated copies of the eTLR system in *AAVS1* treated with M3811, AZD-7648, NU7441, KU-0060648, RS-1, Brefeldin, L755507, or SCR7 pyrazine 24 hours post-transfection. Solely the DNA-PKcs inhibitors M3814, AZD-7648, NU7441, and KU-0060648 showed increased HDR rates compared to a range of compounds that enhance CRISPR-mediated homology-directed repair (HDR).
**Figure 2B** demonstrates that the HDR rates are maximally increased at a concentration of 1uM, while upon treatment with AZD-7648, the HDR rates are increased to a maximum at a concentration as low as 0.5 µM.

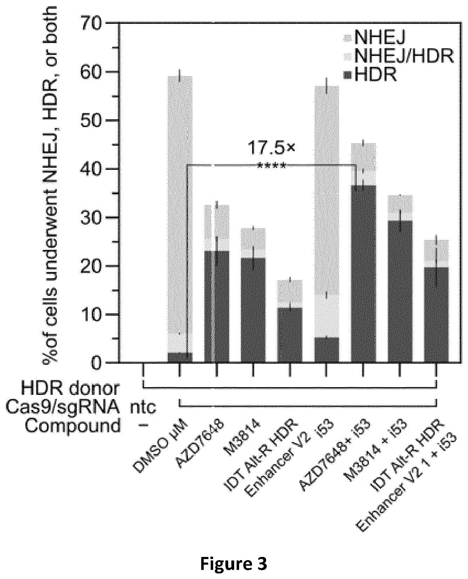

**Figure 3** shows the FACS results 72 h after transfection of HEK293 eTLR cells carrying two stably integrated copies of the eTLR system in *AAVS1* treated with AZD-7648, M3814, ITD Alt-R HDR Enhancer V2, AZD-7648 and the 53BP1 inhibitor (i53BP1 or i53 or i53BP), M3814 and i53BP1, ITD Alt-R HDR Enhancer V2 or i53BP1 four hours prior to transfection. i53, a 53BP1 inhibitor, is co-expressed as a plasmid when indicated. Of note, a combination of AZD-7648 and i53BP1, or M3814 and i53BP1, yields significantly increased HDR rates. In particular, the combination of AZD-7648 and i53BP1 results in a 17.5-fold higher HDR rate than the control-treated with DMSO. Both ITD Alt-R HDR Enhancer V2 alone and a combination of ITD Alt-R HDR Enhancer V2 and i53BP1 showed the smallest effect on HDR rates.

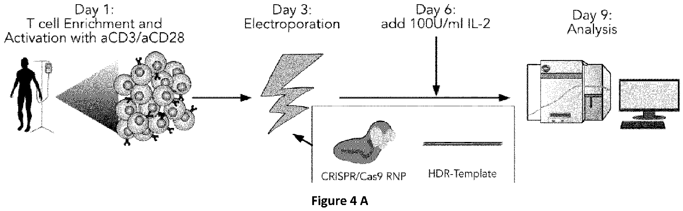

**Figure 4A** shows the workflow used for all experiments: on day 0, cells were either thawed (if obtained and processed earlier) or isolated from buffy coats by gradient centrifugation. The next day (day 1), the human primary T cells were enriched for CD4+ T cells and activated, followed by electroporation with the CRISPR/Cas9 RNR of interest at day 3. IL-2 was added on day 6, and the final analysis by fluorescence-activated cell sorting was performed on day 9.

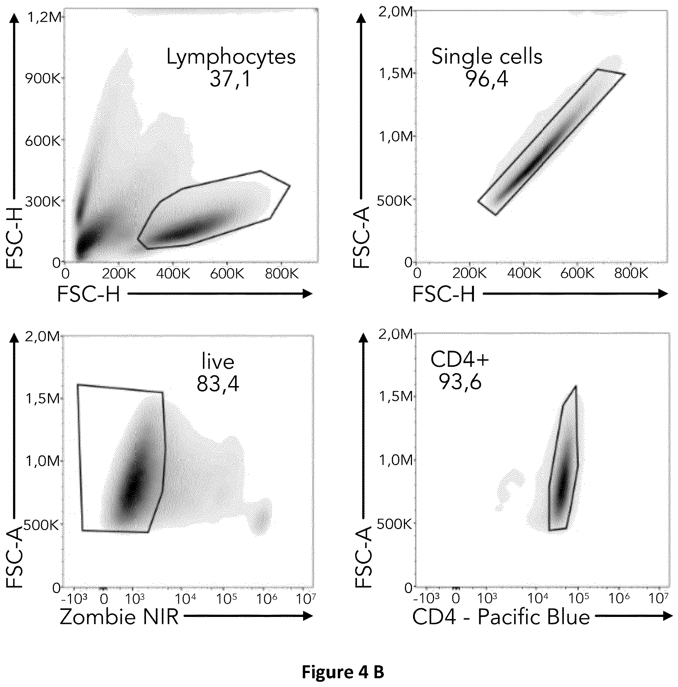

**Figure 4B** demonstrates the gating strategy of the FACS experiments. In each experiment, the events were gated for human T cells first, followed by single-cell gating. Then, the cells were gated for living cells and subsequently for CD4+ cells. The final gate was set for mScarlet-positive cells (red fluorescence).

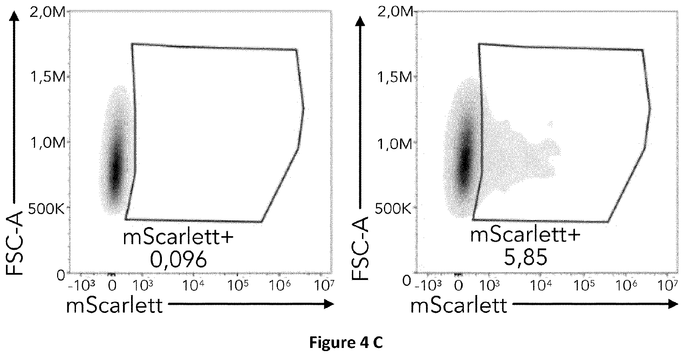

**Figure 4C** shows a representative FACS plot (left) demonstrating the control condition without (ss)DNA, while the right plot shows the human primary T-cells treated with ssDNA + AZD-7648 + i53BP.

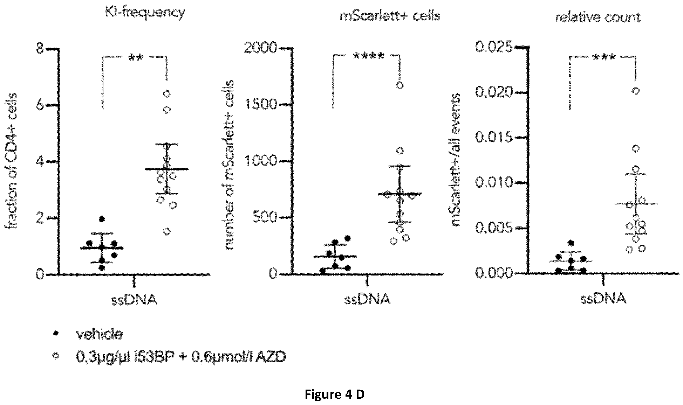

**Figure 4D** displays the knock-in frequency and the potential contribution of the individual components (ssDNA versus dsDNA, AZD7642 and i53BP vs. vehicle and i53BP). Bars indicate the mean with a 95% confidence interval. Black dots indicate vehicle,

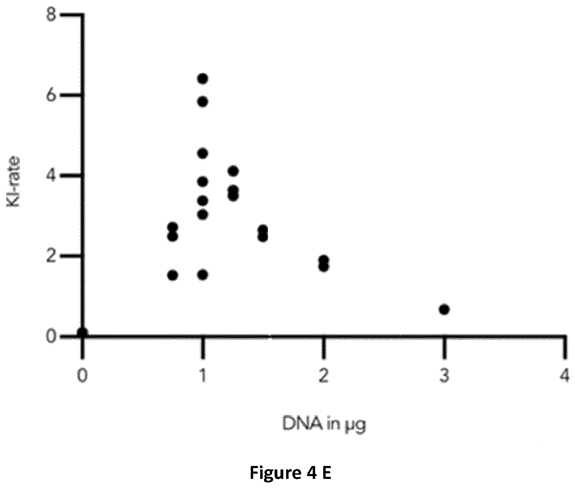

**Figure 4E** shows the results of a titration series of ssDNA tested in knock-in experiments. All titrations were performed in the presence of AZD-7648 and i53BP. The optimal knock-in rate was achieved with 1 µg of ssDNA.

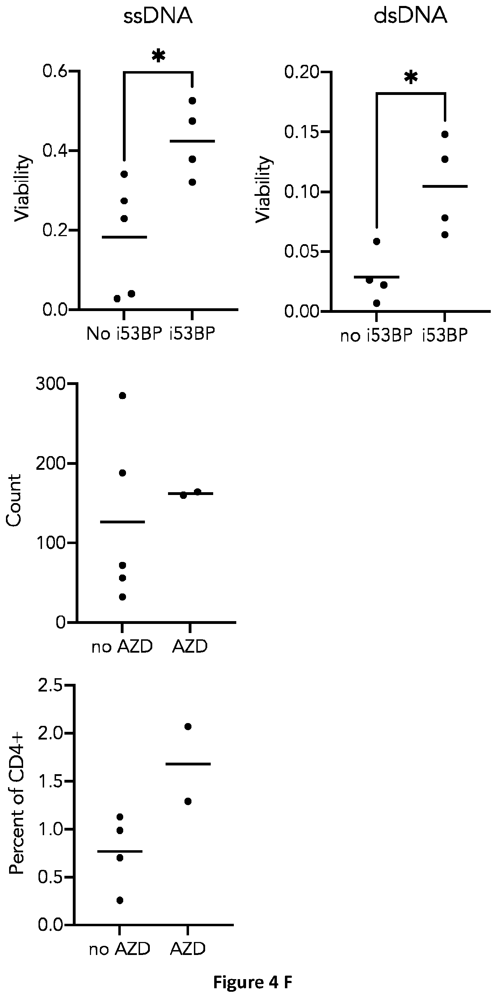

**Figure 4F** shows the effects of i53BP on the viability and the effects of AZD-7648 on the absolute number of edited cells and their respective relative knock-in rate. Viability was calculated as "fraction of cells in the lymphocyte gate" times "fraction of cells in the live gate". The relative knock-in rate refers to the proportion of edited cells in all CD4+ T cells. 1 µg of DNA was used in each experiment, except for the upper right panel, where 1.5 µg was used. Statistical analysis was performed using student's t-test. For the comparisons using AZD-7648, a trend could be observed, but no significant differences. Bars indicate the arithmetic mean. *n* = 2 - 6 biological replicates.

### DETAILED DESCRIPTION OF THE INVENTION

In one aspect, a composition comprising a DNA-dependent protein kinase catalytic subunit inhibitor, and a 53BP1 inhibitor, wherein the DNA-dependent protein kinase catalytic subunit inhibitor is AZD-7648 and/or M3814 and wherein the 53BP1 inhibitor is set forth in SEQ ID NO: 17 is claimed. In one embodiment, the composition comprising a DNA-dependent protein kinase catalytic subunit inhibitor, and a 53BP1 inhibitor results in an additive effect. In another embodiment, the composition comprising a DNA-dependent protein kinase catalytic subunit inhibitor, and a 53BP1 inhibitor yields a synergistic effect. The 53BP1 inhibitor may be a plasmid. The gene editing complex and the 53PB1 inhibitor set forth in SEQ ID NO:17 can be delivered to a cell in the form of DNA, mRNA, proteins, RNPs using different delivery methods, such as AAV, lentiviruses, adenoviruses, or any other suitable virus or lipid nanoparticles (LNPs), liposomes, or any other suitable form of delivery into a host cell or organism. The surface of these lipid nanoparticles may further be equipped with targeting modalities to enhance cellular uptake. These modalities can be optionally combined. In another embodiment, the composition comprises donor nucleic acid molecules. In a preferred embodiment, the composition comprises donor ssDNA or donor dsDNA. The donor ssDNA or donor dsDNA may be amplified *in vitro,* or *in vivo,* or chemically synthesized. The length of the donor ssDNA may be in the range of about 10 to 10000 nucleotides, preferably 20 to 2000 nucleotides, more preferably 500 to 200 nucleotides. In a particularly preferred embodiment, the donor nucleic acid molecule is ssDNA. In even another embodiment, the donor ssDNA is linear ssDNAThe donor ssDNA may be provided in a plasmid, phages, or for example, AAV virus or (other) ssDNA viruses. The donor ssDNA may be provided in a HDR plasmid. The composition may also comprise CRISPR ribonucleoproteins.

Disclosed herein but not part of the invention, variants or homologs of the 53BP1 inhibitor (e.g., variants of SEQ ID NO:28-30) are provided, which are at least about 70% identical, at least about 80% identical, at least about 90% identical, at least about 95% identical, at least about 98% identical, at least about 99% identical, at least about 99.5% identical, or at least about 99.9% to SEQ ID NO:28-30.

In a preferred embodiment, the combination of the DNA-PKcs inhibitor is AZD-7648 and the 53BP1 inhibitor having the sequence set forth in SEQ ID NO:17 yields an additive effect with regards to gene editing events, in particular to HDR events. In an even more preferred embodiment, the combination of the DNA-PKcs inhibitor AZD-7648 and the 53BP1 inhibitor having the sequence set forth in SEQ ID NO:17 yields a synergistic effect with regards to gene editing events, in particular to HDR events. In another preferred embodiment, the combination of the DNA-PKcs inhibitor AZD-7648 and the 53BP1 inhibitor having the sequence set forth in SEQ ID NO:17 yields an approximately 10-fold to approximately 20-fold higher HDR rate compared to control. The combination of the DNA-PKcs inhibitor AZD-7648 and the 53BP1 inhibitor having the sequence set forth in SEQ ID NO:17 may yield a 17.5-fold higher HDR rate compared to control.

In a preferred embodiment, the combination of the DNA-PKcs inhibitor is M3814 and the 53BP1 inhibitor having the sequence set forth in SEQ ID NO: 17 yields in an additive effect with regards to gene editing events, in particular to HDR events. In an even more preferred embodiment, the combination of the DNA-PKcs inhibitor is M3814 and the 53BP1 inhibitor having the sequence set forth in SEQ ID NO:17 yields a synergistic effect with regards to gene editing events, in particular to HDR events.

In another embodiment, the composition comprises both AZD-7648 and M3814, and the 53BP1 inhibitor has the sequence set forth in SEQ ID NO:17. In a preferred embodiment, the DNA-PKcs inhibitor is AZD-7648 and M3814, and the 53BP1 inhibitor having the sequence set forth in SEQ ID NO:17 and said combination yields an additive effect with regards to gene editing events, in particular to HDR events. In an even more preferred embodiment, the DNA-PKcs inhibitor is AZD-7648 and M3814, and the 53BP1 inhibitor having the sequence set forth in SEQ ID NO:17 and said combination yields a synergistic effect with regards to gene editing events, in particular to HDR events. The gene editing events may be read out by an eTLR system, said eTLR system may involve transfection of HEK293 cells carrying two stably integrated copies of the eTLR system in AAVS1Gene editing events may be NHEJ and HDR, or the ratio of NHEJ/HDR. Preferred gene editing events are HDR, in particular CRISPR-mediated HDR.

The composition may comprise both AZD-7648 and M3814 in a range from about 5 wt %: 95 wt%, 10 wt%: 90 wt%, 15 wt%: 85 wt%, 20 wt%: 80 wt%, 25 wt%: 75wt%, 30 wt%: 70wt%, 35wt%: 65 wt%,40 wt%: 60 wt%, 45 wt%: 55wt%, 50wt%: 50wt%,55 wt%: 45wt%, 60 wt%: 40 wt%, 65wt%: 35 wt%,70 wt%: 30wt%, 75 wt%: 25wt%, 80 wt%: 20 wt%, 85 wt%: 15 wt%, 90 wt%: 10 wt%, 95 wt%: 5 wt%, and the 53BP1 inhibitor set forth in SEQ ID NO:17. The composition may comprise both AZD-7648 and M3814 in a range from about 35wt%: 65 wt%,40 wt%: 60 wt%, 45 wt%: 55wt%, 50wt%: 50wt%,55 wt%: 45wt%, 60 wt%: 40 wt%, 65wt%: 35 wt% and the 53BP1 inhibitor has the sequence set forth in SEQ ID NO: 17.

In one embodiment, the composition, including all its combinations, is for use in medicine. In a preferred embodiment, the composition comprises AZD-7648 and the 53BP1 inhibitor set forth in SEQ ID NO: 17 and is for use in medicine. In another preferred embodiment, the composition comprises M3814 and the 53BP1 inhibitor set forth in SEQ ID NO: 17, and said composition is for use in medicine. In yet another preferred embodiment, the composition comprises AZD-7648, M3814 and the 53BP1 inhibitor set forth in SEQ ID NO: 17 and is for use in medicine. In another embodiment, the composition, including all its combinations, is for use as a medicament. In a preferred embodiment, the composition comprises AZD-7648 and the 53BP1 inhibitor set forth in SEQ ID NO: 17 and is for use as a medicament. In another preferred embodiment, the composition comprises M3814 and the 53BP1 inhibitor set forth in SEQ ID NO: 17 and is for use as a medicament. In yet another preferred embodiment, the composition comprises AZD-7648, M3814, and the 53BP1 inhibitor set forth in SEQ ID NO: 17 and is for use as a medicament. In another embodiment, the use in medicine encompasses gene therapy of, for example, a metabolic dysfunction. Gene therapy in the context of the invention means that cells from a patient are gene-edited in the presence of a combination of compounds as described above, whereby both the efficiency of genome editing and the viability of the cells is increased. Not part of the invention but disclosed is that the gene editing method or procedure can be performed *ex vivo* or *in vivo. Ex vivo* means that the isolated cells from the patient are retransferred to the patient after the gene editing procedure. *In vivo* means that the combination as described above is administered to the patient. The combinations (all combinations) according to the invention may be administered alone, i.e., only the combination such as the combination of DNA-Pkcs inhibitor and 53BP1 inhibitor, or in combination with further agents, such as small molecules, antibodies, chemotherapeutic agents. The patient may be a mammalor a human being.

In another embodiment, the composition comprises AZD-7648 and the 53BP1 inhibitor set forth in SEQ ID NO:17 and is for use in the treatment of cancer. In another preferred embodiment, the composition comprises M3814 and the 53BP1 inhibitor set forth in SEQ ID NO:17, and said composition is for use in the treatment of cancer. In yet another preferred embodiment, the composition comprises AZD-7648, M3814, and the 53BP1 inhibitor set forth in SEQ ID NO:17 and is for use in the treatment of cancer. In a preferred embodiment, the composition is for use in the treatment of Acute Lymphoblastic Leukemia (ALL), Acute Myeloid Leukemia (AML), Adrenocortical Carcinoma, Anal Cancer, Astrocytoma, Atypical Teratoid/Rhabdoid Tumor, Basal Cell Carcinoma of the Skin, Cholangiocarcinoma, Bladder Cancer, Bone Cancer (including Ewing Sarcoma, Osteosarcoma and Malignant Fibrous Histiocytoma), Brain Tumors, Breast Cancer, Burkitt Lymphoma, Carcinoid Tumor, Carcinoma of Unknown Primary, Heart Tumors, Medulloblastoma, Germ Cell Tumor, Cervical Cancer, Chordoma, Chronic Lymphocytic Leukemia (CLL), Chronic Myelogenous Leukemia (CML), Chronic Myeloproliferative Neoplasms, Colorectal Cancer, Craniopharyngioma, Cutaneous T-Cell Lymphoma, Endometrial Cancer (Uterine Cancer),Ependymoma, Esophageal Cancer, Head and Neck Cancer, Extracranial Germ Cell Tumor, Childhood Extragonadal Germ Cell Tumor, Retinoblastoma, Fallopian Tube Cancer, Gastric Cancer, Gastrointestinal Carcinoid Tumor, Gastrointestinal Stromal Tumors (GIST), Germ Cell Tumors, Gestational Trophoblastic Disease, Hairy Cell Leukemia, Head and Neck Cancer, Hepatocellular Cancer, Hodgkin Lymphoma, Islet Cell Tumors, Pancreatic Neuroendocrine Tumors, Kaposi Sarcoma, Renal Cell Cancer, Langerhans Cell Histiocytosis, Lung Cancer (Non-Small Cell, Small Cell, Pleuropulmonary Blastoma, and Tracheobronchial Tumor), Melanoma, Merkel Cell Carcinoma (Skin Cancer), Mesothelioma, Multiple Myeloma/Plasma Cell Neoplasms, Mycosis Fungoides, Myelodysplastic Syndromes, Myelodysplastic/Myeloproliferative Neoplasms, Neuroblastoma, Non-Hodgkin Lymphoma, Ovarian Cancer, Pancreatic Cancer, Pancreatic Neuroendocrine Tumors (Islet Cell Tumors), Papillomatosis, Parathyroid Cancer, Penile Cancer, Pheochromocytoma, Pituitary Tumor, Primary Peritoneal Cancer, Prostate Cancer, Rectal Cancer, Rhabdomyosarcoma, Testicular Cancer, Thymoma and Thymic Carcinoma, Thyroid Cancer, Ureter and Renal Pelvis Cancer, Urethral Cancer, Uterine Cancer, Vaginal Cancer, Vulvar Cancer, Wilms Tumor and other childhood renal Tumors.

In another preferred embodiment, the composition comprises AZD-7648 and the 53BP1 inhibitor set forth in SEQ ID NO: 17 and is for use in the treatment of cancer which has been treated with platinum-based compounds, is being treated with platinum-based compounds, or will be treated with platinum-based compounds. In another preferred embodiment, the composition comprises M3814 and the 53BP1 inhibitor set forth in SEQ ID NO: 17 and is for use in the treatment of cancer which has been treated with platinum-based compounds, is being treated with platinum-based compounds, or will be treated with platinum-based compounds. In yet another preferred embodiment, the composition comprises AZD-7648, M3814, and the 53BP1 inhibitor set forth in SEQ ID NO: 17 and is for use in the treatment of cancer which has been treated with platinum-based compounds, is being treated with platinum-based compounds or will be treated with platinum-based compounds.

In a preferred embodiment, the composition comprises AZD-7648 and the 53BP1 inhibitor set forth in SEQ ID NO: 17 and is for use in the treatment of cancer which has been treated with platinum-based compounds, is being treated with platinum-based compounds or will be treated with platinum-based compounds and at least another chemotherapeutic agent such as alkylating agents, anthracyclines, taxanes, topoisomerase inhibitors, kinase inhibitors, nucleotide analogs, and precursors analogs, peptide antibiotics, retinoids, vinca alkaloids and derivatives thereof, therapeutic antibodies and/or cancer immunotherapy. In another preferred embodiment, the composition comprises M3814 and the 53BP1 inhibitor set forth in SEQ ID NO: 17 and is for use in the treatment of cancer which has been treated with platinum-based compounds, is being treated with platinum-based compounds, or will be treated with platinum-based compounds and at least another chemotherapeutic agent such as alkylating agents, anthracyclines, taxanes, topoisomerase inhibitors, kinase inhibitors, nucleotide analogs and precursors analogs, peptide antibiotics, retinoids, vinca alkaloids and derivatives thereof, therapeutic antibodies and/or cancer immunotherapy In yet another preferred embodiment, the composition comprises AZD-7648, M3814 and the 53BP1 inhibitor set forth in SEQ ID NO: 17 and is for use in the treatment of cancer which has been treated with platinum-based compounds, is being treated with platinum-based compounds, or will be treated with platinum-based compounds and at least another chemotherapeutic agent such as alkylating agents, anthracyclines, taxanes, topoisomerase inhibitors, kinase inhibitors, nucleotide analogs and precursors analogs, peptide antibiotics, retinoids, vinca alkaloids and derivatives thereof, therapeutic antibodies and/or cancer immunotherapy. In another preferred embodiment, the platinum-based compounds are, e.g., cisplatin, carboplatin, oxaliplatin, lobaplatin, nedaplatin, picoplatin, satraplatin, triplatin tetranitrate, phenanthriplatin, as well as combinations and derivatives thereof.

In a preferred embodiment, the composition comprises AZD-7648 and the 53BP1 inhibitor set forth in SEQ ID NO: 17 and is for use in the treatment of lung, breast, ovarian, or colon cancer, which has been treated with platinum-based compounds, is being treated with platinum-based compounds, or will be treated with platinum-based compounds. In another preferred embodiment, the composition comprises M3814 and the 53BP1 inhibitor set forth in SEQ ID NO: 17 and is for use in the treatment of lung, breast, ovarian, or colon cancer, which has been treated with platinum-based compounds, is being treated with platinum-based compounds, or will be treated with platinum-based compounds. In yet another preferred embodiment, the composition comprises AZD-7648, M3814, and the 53BP1 inhibitor set forth in SEQ ID NO: 17 and is for use in the treatment of lung, breast, ovarian, or colon cancer which has been treated with platinum-based compounds, is being treated with platinum-based compounds or will be treated with platinum-based compounds.

In another embodiment, the composition is a gene editing enhancing composition, such as a research tool or a tool for gene editing *in vitro, ex vivo,* and *in vivo.* The gene editing enhancing composition does not relate to subject-matter excluded from patentability, such as processes for cloning human beings, processes for modifying the germ line identity of human beings, and uses of human embryos for industrial and commercial purposes. In one embodiment, the gene editing enhancing composition is used in conjunction with gene editing, i.e., a method involving, e.g., a zinc finger nuclease (ZFN), a transcription activator-like effector-based nuclease (TALEN), a CRISPR-Cas system, or combinations thereof. In a preferred embodiment, a CRISPR-Cas system is used.

In another aspect, a gene editing method comprising the sequential steps of: (i) Enriching isolated cells, followed by activating said isolated cells by pre-determined surface antigens; (ii) Adding a composition according to the previous aspect and its embodiments to said cells simultaneously to transfecting said cells by electroporation or transfection reagents two days after step (i); (iii) Adding a stimulant to said cells five days after step (i); (iv) Analyzing the genome editing efficiency and viability of said cells by fluorescent-activated cell sorting is disclosed.

In one embodiment, the isolated cells of step (i) are eukaryotic cells. Eukaryotic cells of the present invention are listed below. The cells can be enriched by various different methods known to the skilled person. For example, enrichment can take place by one or more pre-determined surface antigens, wherein the surface antigens are, for example, members of the cluster of differentiation family, such as, for example, CD2, CD3, CD4, CD8, CD34, CD68, etc. and/or combinations thereof. In one embodiment, the pre-determined surface antigen is a T-cell surface antigen. In another embodiment, the pre-determined surface antigen is CD4. In a preferred embodiment, enrichment takes place by commercially available T-cell enrichment kits, preferably human T-cell enrichment kits. The cells are isolated from, for example, various tissue sources, such as blood, umbilical cords, or any other tissue such as skin tissue. In one embodiment, the enriched T cells are activated by αCD28 and/or αCD3, and/or CD2. In a preferred embodiment, the pre-determined surface antigens are αCD28 and/or αCD3 and the activated cells are human T cells. In a particularly preferred embodiment, the pre-determined surface antigens are αCD28 and αCD3.

In one embodiment, the gene editing method is, for example, CRISPR, TALEN, zinc-finger nucleases, meganucleases, or combinations thereof.

In another embodiment, the DNA-Pkcs inhibitor AZD-7648 or M3814 of the added composition is in the range of 0.01 µM to 10 µM, preferably between 0.1 µM and 5 µM, more preferably between 0.25 µM and 5 µM and most preferably between 0.5 µM and 2.5 µM. In a preferred embodiment, between 0.5 µM and 2 µM of AZD-7648 and/or M3814 is added to the cells. In yet another preferred embodiment, 1 µM of AZD-7648 and/or M3814 is added to the cells. In one embodiment, the DNA-Pkcs inhibitor of the added composition is added to the cells for five minutes up to ten days. In a preferred embodiment, the DNA-Pkcs inhibitor of the added composition is added to the cells for three to seven days. In a particularly preferred embodiment, the DNA-Pkcs inhibitor of the added composition is added to the cells for five to seven days. In another embodiment, the nucleic acids or proteins are transfected into the cells by, e.g., lipofection, electroporation, calcium-phosphate, or virus-based methods. In yet another embodiment, step (ii) takes place 0.5 to five days after step (i). In a preferred embodiment, step (ii) takes place 1 to 3 days after step (ii). In a particularly preferred embodiment, step (ii) takes place two days after step (i).

In yet another embodiment, a stimulant is added to the cells five days after step (i). In one embodiment, the stimulant is, for example, a cytokine. In a preferred embodiment, the stimulant is selected from the interleukin family. In a particularly preferred embodiment, the stimulant is IL-2. In one embodiment, the stimulant is added to the cells three to seven days after step (i). In a preferred embodiment, the stimulant is added to the cells four to six days after step (i). In a particularly preferred embodiment, the stimulant is added to the cells five days after step (i).

In even another embodiment, the genome editing efficiency and viability of the cells are analyzed by fluorescent-activated cell sorting. In another embodiment, the genome editing efficiency and viability of the cells are analyzed by sequencing and cell viability assays such as luminescent cell viability assays.

In one embodiment, the isolated cells of step (i) are eukaryotic cells. In a preferred embodiment, the eukaryotic cells are fungal, yeast, plant, animal, or in some instances, algae cells. In another preferred embodiment, the eukaryotic cells are fungal cells and are selected from the genera *Aspergillus, Penicillium, Acremonium, Trichoderma, Chrysoporium, Mortierella, Kluyveromyces* or *Pichia.* In an even more preferred embodiment, the fungal cell is selected from the species *Aspergillus niger, Aspergillus nidulans, Aspergillus oryzae, Aspergillus terreus, Penicillium chrysogenum, Penicillium citrinum, Acremonium Chrysogenum, Trichoderma reesei, Mortierella alpine, Chrysosporium lucknowense, Kluyveromyces lactis, Pichia pastoris* or *Pichia ciferrii.*

In yet another embodiment, the eukaryotic cells are yeast cells and are selected from the group consisting of the genera *Saccharomyces, Kluyveromnyces,* and *Candida.* In a preferred embodiment, the yeast cell is selected from the group consisting *of Saccharomyces cerevisiae, Saccharomyces uvarum, Kluyveromnyces fragilis, Kluyveromnyces marxianus,* and *Candida utilis.* In even another preferred embodiment, the eukaryotic cells are plant cells and are selected from the group consisting of dicotyledonous plants such as those belonging to the genera *Atropa, Alseodaphne, Anacardium, Arachis, Beilschmiedia, Brassica, Carthamus, Cocculus, Croton, Cucumis, Citrus, Citrullus, Capsicum, Catharanthus, Cocos, Coffea, Cucurbita, Daucus, Duguetia, Eschscholzia, Ficus, Fragaria, Glaucium, Glycine, Gossypium, Helianthus, Hevea, Hyoscyamus, Lactuca, Landolphia, Linum, Litsea, Lycopersicon, Lupinus, Manihot, Majorana, Malus, Medicago, Nicotiana, Olea, Parthenium, Papaver, Persea, Phaseolus, Pistacia, Pisum, Pyrus, Prunus, Raphanus, Ricinus, Senecio, Sinomenium, Stephania, Sinapis, Solanum, Theobroma, Trifolium, Trigonella, Vicia, Vinca, Vitis, and Vigna,* and monocotyledonous plants such as those belonging to the genera *Allium, Andropogon, Aragrostis, Asparagus, Avena, Cynodon, Elaeis, Festuca, Festulolium, Heterocallis, Hordeum, Lemna, Lolium, Musa, Oryza, Panicum, Pannesetum, Phleum, Poa, Secale, Sorghum, Triticum, and Zea;* or the gymnosperm genera *Abies, Cunninghamia, Picea, Pinus,* and *Pseudotsuga.* In a particularly preferred embodiment, the plant cell is selected from the species *Arabidospis thaliana, Nicotiana tabaccum, Solanum lycopersicum, Solanum tuberosum, Solanum melongena, Solanum esculentum, Lactuca saliva, Brassica napus, Brassica oleracea, Brassica rapa, Oryza glaberrima, Oryza sativa, Asparagus officinalis, Pisum sativum, Medicago sativa, Zea mays, Hordeum vulgare, Secale cereal, Triticum aestivum, Triticum durum, Capsicum sativus, Cucurbita pepo, Citrullus lanatus, Cucumis melo, Citrus aurantifolia, Citrus maxima, Citrus medica,* or *Citrus reticulata.*

In yet another embodiment, the eukaryotic cells are animal cells and are selected from the group consisting of the genera Homo, Rattus, Mus, Sus, Bos, Danio, Canis, Felis, Equus, Salmo, Oncorhynchus, Gallus, Meleagris, Drosophila, or Caenorhabditis. In a preferred embodiment, the animal cell can be of the species *Homo sapiens, Rattus norvegicus, Mus musculus, Sus scrofa, Bos taurus, Danio rerio, Salmo salar, Canis lupus, Felis catus, Equus caballus, Oncorhynchus mykiss, Gallus gallus, Meleagris gallopavo, Drosophila melanogaster* or *Caenorhabditis elegans.* The animal cell can be from a cow, pig, sheep, goat, bison, horse, donkey, mule, rabbit, chicken, duck, goose, turkey, or pigeon. In a particularly preferred embodiment, the animal cell is a mammalian cell and is selected from the group consisting of neuronal cells, glial cells, cardiomyocytes, glucose-responsive insulin-secreting pancreatic beta cells, hepatocytes, astrocytes, oligodendrocytes, chondrocytes, osteoblasts, retinal pigment epithelial cells, fibroblasts, keratinocytes, dendritic cells, hair follicle cells, renal duct epithelial cells, vascular endothelial cells, testicular progenitors, smooth and skeletal muscle cells, hematopoietic cells, cartilage cells or epidermal cells as well as insulin-secreting β-cells, embryonic stem cells, and mesenchymal cells. In an even more preferred embodiment, the mammalian cell is a human cell and is selected from the group consisting of neuronal cells, glial cells, cardiomyocytes, glucose-responsive insulin-secreting pancreatic beta cells, hepatocytes, astrocytes, oligodendrocytes, chondrocytes, osteoblasts, retinal pigment epithelial cells, fibroblasts, keratinocytes, dendritic cells, hair follicle cells, renal duct epithelial cells, vascular endothelial cells, testicular progenitors, smooth and skeletal muscle cells, hematopoietic cells, cartilage cells or epidermal cells as well as insulin-secreting β-cells, embryonic stem cells, and mesenchymal cells. In yet another embodiment, the eukaryotic cells are algae cells and are selected from the genera *Laminaria, Saccharina, Ectocarpus, Plamaria, Gracilaria, Porphyra, Ulva,* or *Enteromporpha.* In a preferred embodiment, the isolated cells of step (i) are human stem cells. In a preferred embodiment, the human stem cells are adult stem cells or induced pluripotent stem cells. In an even more preferred embodiment, the adult stem cells are hematopoietic stem cells, umbilical cord blood stem cells, amniotic fluid stem cells, mesenchymal stem cells, neural stem cells, or epithelial stem cells. In an even more preferred embodiment, the isolated cells of step (i) are human hematopoietic stem cells.

In yet another embodiment, a site-specific insertion of a cassette comprising between 1 and 10,000 nucleotides into a pre-defined locus takes place in steps (ii) and (iii). In a preferred embodiment, the site-specific insertion of a cassette comprises between 2000 and 10,000 nucleotides. In even another embodiment, the genome editing efficiency of step (iv) is improved by at least 2-fold and at most improved by 600-fold compared to genome editing methods in which no composition as described in the previous aspect and its embodiments is added to the cells.

In a preferred embodiment, the genome editing efficiency of step (iv) is improved by at least 10-fold compared to genome editing methods in which no composition as described in the previous aspect and its embodiments is added to the cells. In a particularly preferred embodiment, the genome editing efficiency of step (iv) is improved by approximately 17.5-fold compared to genome editing methods in which no composition as described in the previous aspect and its embodiments is added to the cells. Improved genome editing efficiency in the context of the present invention means that the HDR rate is increased by a factor "x," for example, by 17.5-fold compared to a negative control.

Disclosed but not part of the invention is an engineered cell obtained by the gene editing method of the previous aspect, wherein the cell is a human stem cell. The cell may be a human hematopoietic stem cell,a CAR T-cell, a regulatory T cell, a helper T cell, a cytotoxic T cell, or a NK cell.

Also disclosed but not part of the invention is the use of the composition in gene editing. The use of the composition in gene editing may be an *in vivo, ex vivo,* or *in vitro* use. The use does not relate to subject-matter excluded from patentability, such as processes for cloning human beings, processes for modifying the germ line identity of human beings and uses of human embryos for industrial and commercial purposes.

### EXAMPLES

### Example 1: Enhanced traffic light reporter system (eTLR)

Figure 1 shows the principle of an "enhanced traffic light reporter" (eTLR) system reporting both mutagenic end-joining events (green fluorescence), such as non-homologous end-joining (NHEJ) or microhomology-mediated end-joining (MMEJ) and homology-directed repair (HDR) events (red fluorescence) after the introduction of a double-strand break (DSB), e.g., by CRISPR/Cas9. Briefly, the system comprises a C-terminally truncated mScarlet-I red fluorescent protein followed by a stop codon and three downstream eUnaG coding sequences, each in one of the respective translational frames. Also, all out-of-frame stop codons that lead to a premature translational termination were removed via silent mutagenesis to activate one of the eUnaG frames after a mutagenic end-joining event. The mutation T52S was introduced into eUnaG to remove an out-of-frame stop codon that could not be removed silently otherwise. Moreover, all out-of-frame ATGs were also removed, which possibly lead to a cryptic translational initiation and thus the cryptic activation of one of the eUnaG frames. The HDR donor template contains a promoterless ATG-less full coding frame of mScarlet-I and a polyadenylation site with 5' and 3' homology arms (HA). HDR-repair leads to the repair of the mScarlet-I reporter and, therefore, the expression of the mScarlet I red fluorescent protein, while mutagenic end-joining events, such as NHEJ and MMEJ, lead to the translation of one of the three green fluorescent eUnaG proteins. If desired, immunofluorescence can decode the exact frame by staining one of the three epitope tags (FLAG, HA, V5).

### Example 2: DNA-Pkcs inhibitor studies in HEK293 eTLR cells

### Cell lines and maintenance of HEK293 eTLR

HEK293T eTLR cell lines were maintained in an H₂O-saturated atmosphere in Gibco Advanced DMEM (Thermo Fisher Scientific) supplemented with 10% FBS (Gibco, Thermo Fisher Scientific), GlutaMax (Gibco, Thermo Fisher Scientific), and penicillin-streptomycin (Gibco, Thermo Fisher Scientific) at 100 µg ml-1 at 37 °C and 5% CO₂. Cells were passaged at 90% confluency by removing the medium, washing with DPBS (Gibco, Thermo Fisher Scientific), and separating the cells with 2.5 ml of Accutase solution (Gibco, Thermo Fisher Scientific). Cells were then incubated for five to ten minutes at room temperature until a visible detachment of the cells was observed. Accutase was subsequently inactivated by adding 7.5 ml pre-warmed DMEM, including 10% FBS and all of the supplements. Cells were then transferred into a new flask at an appropriate density or counted and plated in 48-well (75,000 cells in 500 µl medium per well) for plasmid transfection.

### Plasmid transfection

Cells were transfected with X-tremeGENE HP (Roche) or jetOPTIMUS (Polyplus Transfection) according to the manufacturer's protocol. DNA amounts were kept constant in all of the transient experiments to yield reproducible complex formation and comparable results. In a 48-well-plate experiment, a total amount of 300 ng of plasmid DNA was used per well. Cells were plated one day before transfection (75,000 cells per well) in 500 µl in a 48-well plate.

### Small-molecule manipulation of DNA repair pathways in HEK293 eTLR cell line

For modulation of DNA repair pathways in the HEK293 eTLR cell line, the compounds (M3818, AZD-7648, NU7441, KU-0060648, RS-1, Brefeldin, L755507, SCR7 pyrazine) were added 24 hours post-transfection to the cells. Control cells received the same volume of DMSO. HEK293 eTLR cells were transfected in a 48-well plate with the indicated plasmids containing the plasmid DNA donor or the CRISPR/Cas9 expression plasmid targeting the eTLR locus. 72 h after transfection, cells were detached using Accutase (Gibco, Thermo Fisher Scientific), pelleted (200 r.c.f. for 5 min), and resuspended in 200 µl of ice-cold DPBS (Gibco, Thermo Fisher Scientific) containing 2% FBS (Gibco, Thermo Fisher Scientific) for FACS analysis. FACS analysis was performed using the BD FACSaria II system (controlled by the BD FACSDiva software (v.6.1.3, BD Biosciences).

### DNA-Pkcs inhibitor studies in HEK293 eTLR cells

HEK293 eTLR cells (i.e. carrying two stably integrated copies of the eTLR system in AAVS1) were treated with M3811, AZD-7648, NU7441, KU-0060648, RS-1, Brefeldin, L755507, or SCR7 pyrazine 24 hours post-transfection, followed by FACS analysis 72 hours after transfection, Figure 2A. Solely the DNA-PKcs inhibitors M3814, AZD-7648, NU7441, and KU-0060648 showed increased HDR rates compared to a range of compounds that enhance CRISPR-mediated homology-directed repair (HDR). In particular, the tested compounds were RS-1 (Rad51 inhibitor, enhances CRISPR-mediated homology-directed repair (HDR), Brefeldin (lactone antibiotic and ATPase inhibitor which also enhances CRISPR-mediated homology-directed repair (HDR), L755507 (selective β3 adrenergic receptor partial agonist which also enhances CRISPR-mediated homology-directed repair (HDR), or SCR7 pyrazine (enhances CRISPR-mediated homology-directed repair (HDR) up to 19-fold while also inhibiting non-homologous end-joining (NHEJ).

The optimal concentration of the two DNA-PKcs inhibitors M3814 and AZD-7648 was determined by testing a range of concentrations; 0.5 to 2 µM in the case of M3814 µM and 0.5 to 2 µM in the case of AZD-7648. 72 h after transfecting the HEK293 eTLR cells (i.e., carrying two stably integrated copies of the eTLR system in AAVS1), said HEK293 eTLR cells were treated with a range of M3814 or AZD-7648, Figure 2B. Upon treatment with M3814, the HDR rates are maximally increased at a concentration of 1uM, while upon treatment with AZD-7648, the HDR rates are maximally increased at a concentration of 0.5 µM already, Figure 2B.

### Example 3: Combination of DNA-Pkcs inhibition and 53BP1 inhibition in HEK293 eTLR cells

### Cell lines and maintenance of HEK293 eTLR

HEK293T eTLR cell lines were maintained in an H₂O-saturated atmosphere in Gibco Advanced DMEM (Thermo Fisher Scientific) supplemented with 10% FBS (Gibco, Thermo Fisher Scientific), GlutaMax (Gibco, Thermo Fisher Scientific), and penicillin-streptomycin (Gibco, Thermo Fisher Scientific) at 100 µg ml-1 at 37°C and 5% CO₂. Cells were passaged at 90% confluency by removing the medium, washing with DPBS (Gibco, Thermo Fisher Scientific), and separating the cells with 2.5 ml of Accutase solution (Gibco, Thermo Fisher Scientific). Cells were then incubated for five to ten minutes at room temperature until a visible detachment of the cells was observed. Accutase was subsequently inactivated by adding 7.5 ml pre-warmed DMEM, including 10% FBS and all of the supplements. Cells were then transferred into a new flask at an appropriate density or counted and plated in 48-well (75,000 cells in 500 µl medium per well) for plasmid transfection.

### Plasmid transfection

Cells were transfected with X-tremeGENE HP (Roche) or jetOPTIMUS (Polyplus Transfection) according to the manufacturer's protocol. DNA amounts were kept constant in all of the transient experiments to yield reproducible complex formation and comparable results. In a 48-well-plate experiment, a total amount of 300 ng of plasmid DNA was used per well. Cells were plated 1 d before transfection (75,000 cells per well) in 500 µl in a 48-well plate.

72 hours after transfecting the HEK293 eTLR cells (i.e., carrying two stably integrated copies of the eTLR system in AAVS1), said HEK293 eTLR cells were treated with AZD-7648, M3814, ITD Alt-R HDR Enhancer V2, AZD-7648 and i53BP1, M3814 and i53BP1, ITD Alt-R HDR Enhancer V2 and i53BP1 four hours before transfection, Figure 3. i53, a 53BP1 inhibitor, is co-expressed as a plasmid when indicated. Both AZD-7648 and M3814 alone lead to significantly higher HDR rates than ITD Alt-R HDR Enhancer V2, Figure 3. Of note, a combination of AZD-7648 and i53BP1, or M3814 and i53BP1 yields significantly increased HDR rates, in particular, the combination ofAZD-7648 and i53BP1 results in a 17.5-fold higher HDR rate than the control-treated with DMSO, Figure 3. While the combination of ITD Alt-R HDR Enhancer V2 and i53BP1 increased the HDR rate compared to ITD Alt-R HDR Enhancer V2 alone, both ITD Alt-R HDR Enhancer V2 alone and a combination of ITD Alt-R HDR Enhancer V2 and i53BP1 showed the smallest effect on HDR rates.

### Example 4: Isolation and culture of primary human T-cells

Buffy coats were collected by the German Heart Center Munich, with the approval of the local institutional review board (Ethics Committee TUM School of Medicine, Technical University Munich) and informed consent of all participants or bought from the "Bayerisches Rotes Kreuz München". Buffy coats were processed on day 0 with either Pancoll (PanBiotech) or Lymphoprep (Stem Cell), or Roti-Sep (Carl Roth) using gradient centrifugation in SepMate 50 tubes (Stem Cell). Initial centrifugation at 1200g for 20min. After that, the supernatant was carefully transferred into new falcons and washed with EasySep buffer (PBS with 2% FBS and 1 mmol l^-1 EDTA). The freshly obtained PBMCs were then suspended in complete Roswell Park Memorial Institute (cRPMI), consisting of RPMI 1640 (ThermoFisher) supplemented with 5 mmol l^-1 HEPES (ThermoFisher), 2 mmol l^-1 glutamine (ThermoFisher), 50 µg ml^-1 penicillin/streptomycin (ThermoFisher), 5 mmol l^-1 nonessential amino acids (ThermoFisher), 5 mmol/l sodium pyruvate (ThermoFisher) and 10 % FBS (ThermoFisher). After overnight cultivation (at 37 °C with 5 % CO₂), PBMCs were enriched for CD4+ T-cells on day 1 using a MojoSort Human CD4 T Cell Isolation Kit (Biolegend). The isolated T cells were resuspended with cRPMI with 5 µg ml^-1 αCD28 (Biolegend) and 100 U ml^-1 IL-2 (Peprotech) at a concentration of 1x10⁶ cells ml^-1 and then activated on a 48 well plate, which had been coated overnight with 10 µg ml^-1 *α*CD3 (clone: UCHT1 from Biolegend), for 48 h before electroporation at 37 °C. 0.6 µmol l^-1 AZD-7648 (MedChemExpress) was added to the cell suspension on day 3 five hours prior to electroporation. On day 6, 100 U ml^-1 IL-2 was added to the cultured cells.

### Example 5: Cas9 RNP assembly and generation of knock-in T-cells

80 µM crRNA (IDT) with the sequence 5'-CCTCTAAGGTTTGCTTACGA-3' and 80 µM tracrRNA (IDT) were mixed in a 1:1 ratio and incubated for 5 min at 96 °C to generate 40 µM crRNA-trans-activating CRISPR RNA duplexes. An equal volume of 40 µM *Streptococcus* pyogenesCas9-NLS (made in-house) was carefully added to the crRNA-tracrRNA and incubated for 15 min at 37 °C to generate 20 µM Cas9 RNPs. For each 96-well reaction cuvette (LONZA), 1x10⁶ cells treated with AZD-7648 beforehand were resuspended in 20 µl P3 buffer. 4 µl containing 20 µM Cas9 RNP, 3 µl of i53BP with a concentration of 2 µg µl^-1, and 1 µg - 1.5 µg Homology-directed-repair-template (HDRT) were added to the reaction cuvette, followed by 20 µl P3 buffer containing the cells. CD4+ T cells were electroporated using program EH-115 on the Amaxa 4D-Nucleofector (LONZA). After electroporation, 80 µl cRPMI was added to the reaction cuvette, and the cells were incubated for 30 min at 37 °C before transferring them to a 96 well plate, prepared with 100 µl cRPMI containing 200 U ml^-1 IL-2 (for a final concentration of 100 U ml^-1), 5 µl ImmunoCult Human CD3/CD28/CD2 T Cell Activator (Stem Cell) and 1.2 µmol l^-1 AZD-7648 (for a final concentration of 0.6 µmol l^-1). The cells were then incubated at 37 °C.

### Example 6: Generation of donor dsDNA or donor ssDNA for nucleofection

### Donor dsDNA

A polymerase chain reaction was performed using HiFi iProof Master Mix (BioRad) to generate dsDNA from a plasmid containing the desired sequence. Primers were used at a concentration of 0.5 µmol l^-1, and 10 ng of plasmid DNA was added to 50 µl reaction volume (The usual reaction volume for this PCR was 1200 µl). The PCR product was checked for the correct length by gel electrophoresis using a 1.5 % agarose gel and subsequently purified using Solid Phase Reversible Immobilization beads (SPRI-beads) at a 1:1 ratio. The final concentration was about 1 µg µl^-1 of dsDNA.

### Donor ssDNA

Single-stranded primer deoxyribonucleotides (where one is 5'-phosphorylated) were diluted to 100 µM in nuclease-free water (Integrated DNA Technology (IDT)). PCR reactions with plasmid DNA template containing the DNA donor were performed using the Q5 Hot Start High-Fidelity 2× Master Mix (New England Biolabs (NEB)) according to the manufacturer's protocol. A small aliquot of the PCR reaction was loaded on a DNA agarose gel electrophoresis together with a 1 kb Plus DNA Ladder (NEB) to confirm the PCR product size. After validation, the PCR reactions were purified by using the Monarch DNA & PCR Cleanup Kit (NEB). The PCR product was treated with Lambda exonuclease (NEB) to generate ssDNA according to the manufacturer's protocol, which degraded the 5'-phosphorylated strand only, leaving the other DNA strand intact. The ssDNA was again purified using the Monarch DNA & PCR Cleanup Kit (NEB) and eluted in nuclease-free ddH₂O.

### Example 7: Combination of DNA-Pkcs inhibition and 53BP1 inhibition as well as combination of DNA-Pkcs inhibition, 53BP1 inhibition, and donor ssDNA or donor dsDNA in primary human T cells

### General workflow

Human T cells isolated from subjects were either thawed (if collected earlier) or isolated from buffy coats by gradient centrifugation, following standard protocols on day 0. The next day (day 1), the human T cells were enriched for CD4+ T cells by using a MojoSort Human CD4 T Cell Isolation Kit (Biolegend) and activated with αCD3/αCD28 as described above in Example 4. On day 3, the T-cells were electroporated with the CRISPR/Cas9 RNR of interest as well as the HDR template. 100 U ml^-1 IL-2 was added on day 6, and the final analysis by fluorescence-activated cell sorting was performed on day 9 (Figure 4A).

### FACS analysis of CD4+ cells for HDR events

On day 9, the cells were washed with PBS and then stained for 30 min at 4 °C with *α*CD4-PacificBlue (clone SK3, Biolegend) and Zombie NIR Fixable live/dead (Biolegend). Successfully edited cells were determined by comparing the fraction of mScarlett+ positive cells (a fluorescent protein encoded on the HDRT) of our edited cells to the fraction of mScarlett+ cells in a DNA or non-targeting control (both prevent any incorporation of the DNA template into the genomic DNA of the cells). The cells were analyzed on a Cytoflex LX (Beckman Coulter) with CytExpert acquisition software (Beckman- Coulter).

To investigate and quantify the human primary T cells of interest, i.e., the human primary T cells harboring the desired knock-in, the following gating strategy was used in each experiment. First, the events were gated for human T cells by applying forward scatter height (FSC-H) versus side scatter height (SSC-H) such that the human T cells are identified based on size and granularity. (Figure 4B, left-most plot, "lymphocytes 37,1"). Then, a forward scatter height (FSC-H) versus side scatter height (SSC-H) analysis was applied to exclude doublet cells (single-cell gating, Figure 4B, second plot on the left, "single cells 96,4"). Subsequently, the cells were gated for living cells by applying the Zombie NIR stain versus the forward scatter area (FSC-A, Figure 4B, second plot on the right, "live 83,4") and the desired CD4+ cells by applying CD4 Pacific Blue stain versus the forward scatter area (FSC-A, Figure 4B, right-most plot, "CD4+ 93,6"). The final gate was set for mScarlet-positive cells (red fluorescence).

Representative FACS plots in which the gate is set for Scarlet-positive cells (red fluorescence) versus the forward scatter area (FSC-A) are shown in Figure 4C. The left plot shows the control condition without ssDNA while the right plot shows the T-cells treated with ssDNA, AZD-7648, and i53BP.

The knock-in frequency and the potential contribution of the individual components (ssDNA versus dsDNA, AZD7642 and i53BP vs. no AZD7642 and i53BP) was investigated in Table 1 below and Figure 4D. Table 1 shows a two-way ANOVA to evaluate the contribution of the individual components (ssDNA versus dsDNA, AZD7642 and i53BP vs no AZD7642 and i53BP) and their interaction on the knock-in frequency.

**Table 1: Two-way ANOVA**

| Source of Variation | % of total variation | P value | summary |
|---|---|---|---|
| Interaction | 20,04 | 0,0002 | *** |
| ssDNA vs. dsDNA | 8,653 | 0,0099 | ** |
| no compound vs. compound | 22,5 | 0,0001 | *** |

In general, there is an increase in the number of successfully edited cells upon the use of AZD-7648 and i53BP (Figure 4D, bars indicate the mean with a 95% confidence interval). This increase is independent of the quantification method used; (i) fraction of mScarlet+ cells of all CD4+ cells (Figure 4D, left-most plot); (ii) absolute number of mScarlet+ cells (Figure 4B, plot in the middle), (iii) mScarlet+ cells/all recorded events (Figure 4B, right-most plot).

To optimize the amount of ssDNA further since the amount of DNA is critical in knock-in experiments with respect to the efficiency of the knock-in and impacts cell survival, different amounts of ssDNA were titrated in the knock-in experiments Figure 4E. All titrations were performed in the presence of AZD-7648 and i53BP. The optimal knock-in rate was achieved with 1 µg of ssDNA (per 10^6 cells).

**TABLE OF SEQUENCES DISCLOSED**

| **Sequence #** | **Name** | **Mutation** | **Used in Fig. / Comment** |
|---|---|---|---|
| 1 | DNA encoding DNA-dependent RNA polymerase III promoter U6 (pU6) | | 1-3 |
| 2 | DNA encoding DNA-dependent RNA polymerase II promoter CAG (pCAG) | | 1-3 |
| 3 | DNA encoding DNA-dependent RNA polymerase II promoter CBh (pCBh) | | 1-3 |
| 4 | DNA encoding bGH-pA-Bbsldel; bovine growth hormone (presomatotropin) polyadenylation signal with removed Bbsl restriction site | | 1-3 |
| 5 | DNA encoding bGH-pA; bovine growth hormone (presomatotropin) polyadenylation signal | | 1-3 |
| 6 | DNA encoding sgRNA scaffold with polyU stretch removed v2 and stabilized 1st hairpin | | |
| 7 | RNA encoding sgRNA scaffold with polyU stretch removed v2 and stabilized 1st hairpin | | |
| 8 | DNA encoding G+20-nt-spacer v1 for eTLR system | | 1-3 |
| 9 | RNA encoding G+20-nt-spacer v1 for eTLR system | | |
| 10 | DNA encoding 20-nt spacer for targeting AAVS1 | | 4 |
| 11 | RNA encoding 20-nt spacer for targeting AAVS1 | | |
| 12 | DNA encoding AAVS1-5'-HA | | 4 |
| 13 | DNA encoding AAVS1-3'-HA | | 4 |
| 14 | DNA encoding SFFV-promoter | | 4 |
| 15 | DNA encoding eTLR donor/template for homologous recombination | | 1-3 |
| 16 | DNA encoding donor template used for knock-in of a red membrane protein into CD8+ T-cells: AAVS1-5'-HA_SFFV-promoter_Gaussia-luciferase-signal-peptide:3xP2-coils:4xGGGGS:GYPA-TMD(G102L):mScarlet-I:PMTS_bGH-pA_AAVS1-3'-HA | | 4 |
| 17 | DNA encoding pCAG_i53_bGH-pA | | 3 |
| 18 | DNA encoding pU6_G+20-nt-spacer v1 for eTLR system_sgRNA scaffold with polyU stretch removed v2 and stabilized 1st hairpin_6xT | | 1-3 |
| 19 | DNA encoding pC Bh_SpyCas9:HA:2xSV40NLS_bGH-pA-BbsIdel | | 1-3 |
| 20 | DNA encoding SpyCas9 v1 | | 1-4 |
| 21 | DNA encoding SpyCas9 v2 | | |
| 22 | Amino acid for SpyCas9 | | |
| 23 | DNA encoding for SV40 NLS v1 | | 1-4 |
| 24 | DNA encoding for SV40 NLS v2 | | |
| 25 | Amino acid for SV40 NLS | | |
| 26 | DNA encoding for HA epitope tag (HA) | | 1-3 |
| 27 | Amino acid for HA epitope tag (HA) | | |
| 28 | DNA encoding for i53/i53BP alias ubiquitin Q2L, I44A, Q29S, Q62L, E64D, T66K, L69P, V70L, G75Δ, G76Δ (mammalian codon-optimized) | | 3,4 |
| 29 | DNA encoding for i53/i53BP alias ubiquitin Q2L, I44A, Q29S, Q62L, E64D, T66K, L69P, V70L, G75Δ, G76Δ (E.coli codon-optimized) | | |
| 30 | Amino acid for i53/i53BP alias ubiquitin Q2L, I44A, Q29S, Q62L, E64D, T66K, L69P, V70L, G75Δ, G76Δ | | |
| 31 | DNA encoding for 6xHis (His-tag) | | 4 |
| 32 | Amino acid for 6xHis (His-tag) | | |
| 33 | DNA encoding for TEV-protease cleavage site | | 4 |
| 34 | Amino acid for TEV-protease cleavage site | | |
| 35 | DNA encoding for Gaussia-luciferase-signal-peptide | | 4 |
| 36 | Amino acid for Gaussia-luciferase-signal-peptide | | |
| 37 | DNA encoding for 3xP2-coils | | 4 |
| 38 | Amino acid for 3xP2-coils | | |
| 39 | DNA encoding for 4xGGGGS | | 4 |
| 40 | Amino acid for 4xGGGGS | | |
| 41 | DNA encoding for GYPA transmembrane domain (GYPA-TMD(G102L); with G102L mutation to inactive TMD dimerization)) | | 4 |
| 42 | Amino acid for GYPA transmembrane domain (GYPA-TMD(G102L)) | G102L | To inactivate TMD dimerization |
| 43 | DNA encoding for mScarlet-I | | 4 |
| 44 | Amino acid for mScarlet-I | | |
| 45 | DNA encoding for Plasma-membrane-trafficking-signal (PMTS) | | 4 |
| 46 | Amino acid for Plasma-membrane-trafficking-signal (PMTS) | | |
| 47 | DNA encoding for SpyCas9:HA:2xSV40NLS | | 2, 3/used in all plasmid transfections in HEK293T cells |
| 48 | Amino acid for SpyCas9:HA:2xSV40NLS | | |
| 49 | DNA encoding for His-tag:TEV-protease cleavage site:SpyCas9:2xSV40NLS | | 4/recombinant protein was used in all primary CD4+ T-cells |
| 50 | Amino acid for His-tag:TEV-protease cleavage site:SpyCas9:2xSV40NLS | | |
| 51 | DNA encoding for His-tag:TEV-protease cleavage site:i53 | | 4/ |
| 52 | Amino acid for His-tag:TEV-protease cleavage site:i53 | | |
| 53 | DNA encoding for eTLR coding sequence | | 1-3/contains frameshift sides. All three frames play a role |
| 54 | Amino acid for eTLR coding sequence | | aa(+1) |
| 55 | Amino acid for eTLR coding sequence | | aa(+2) |
| 56 | Amino acid for eTLR coding sequence | | aa(+3) |

### REFERENCES

CANNY, M.D. Inhibition of 53BP1 favors homology-dependent DNA repair and increases
CRISPR-Cas9 genome-editing efficiency. Nat Biotechnol. January 2018, Vol.36, pages 95-102.

## Claims

1. A composition comprising a DNA-dependent protein kinase catalytic subunit inhibitor, and a 53BP1 inhibitor, wherein the DNA-dependent protein kinase catalytic subunit inhibitor is AZD-7648 and/or M3814 and wherein the 53BP1 inhibitor is set forth in SEQ ID NO:17.

2. The composition according to claim 1, wherein the composition comprises donor ssDNA or donor dsDNA.

3. The composition according to any one of claims 1 or 2, wherein the composition comprises donor ssDNA.

4. The composition according to any one of claims 1 to 3 for use in medicine.

5. The composition according to any one of claims 1 to 3, wherein the composition is a gene editing enhancing composition.

6. A gene editing method comprising the sequential steps of:
(i) Enriching isolated cells, followed by activating said isolated cells by pre-determined surface antigens;
(ii) Adding a composition according to any one of claims 1-3 or claim 5 to said cells, simultaneously to transfecting said cells two days after step (i);
(iii) Adding a stimulant to said cells five days after step (i);
(iv) Analyzing the genome editing efficiency and viability of said cells by fluorescent-activated cell sorting.

7. The gene editing method according to claim 6, wherein the isolated cells of step (i) are eukaryotic cells.

8. The gene editing method according to claim 6 or claim 7, wherein the isolated cells of step (i) are human stem cells, preferably human hematopoietic stem cells.

9. The gene editing method according to any one of claims 6 to 8, wherein in step (ii) and (iii) a site-specific insertion of a cassette comprising between 1 and 10,000 nucleotides, preferably between 2000 and 10,000 nucleotides into a pre-defined locus takes place.

10. The gene editing method according to any one of claims 6 to 9, wherein the genome editing efficiency of step (iv) is improved by at least 10-fold compared to genome editing methods, wherein in said genome editing methods no composition according to any one claims 1 to 5 or according to claim 7 is added to the cells.

## Patentansprüche

1. Eine Zusammensetzung, umfassend einen DNA-abhängigen Inhibitor der katalytischen Untereinheit der Proteinkinase und einen 53BP1-Inhibitor umfasst, wobei der DNAabhängige Inhibitor der katalytischen Untereinheit der Proteinkinase AZD-7648 und/oder M3814 ist und wobei der 53BP1-Inhibitor in SEQ ID NO:17 angegeben ist.

2. Die Zusammensetzung nach Anspruch 1, wobei die Zusammensetzung Donor-ssDNA oder Donor-dsDNA umfasst.

3. Die Zusammensetzung nach einem der Ansprüche 1 oder 2, wobei die Zusammensetzung Donor-ssDNA umfasst.

4. Die Zusammensetzung nach einem der Ansprüche 1 bis 3 zur Verwendung in der Medizin.

5. Die Zusammensetzung nach einem der Ansprüche 1 bis 3, wobei die Zusammensetzung eine Genbearbeitung verbessernde Zusammensetzung ist.

6. Ein Genbearbeitungsverfahren, das die folgenden aufeinanderfolgenden Schritte umfasst:
(i) Anreicherung isolierter Zellen, gefolgt von der Aktivierung der isolierten Zellen durch vorbestimmte Oberflächenantigene;
(ii) Hinzufügen einer Zusammensetzung nach einem der Ansprüche 1 bis 3 oder Anspruch 5 zu den Zellen, gleichzeitig mit der Transfektion der Zellen zwei Tage nach Schritt (i);
(iii) Hinzufügen eines Stimulans zu den Zellen fünf Tage nach Schritt (i);
(iv) Analysieren der Genombearbeitungseffizienz und Lebensfähigkeit der Zellen durch fluoreszenzaktivierte Zellsortierung.

7. Das Genbearbeitungsverfahren nach Anspruch 6, wobei die isolierten Zellen aus Schritt (i) eukaryotische Zellen sind.

8. Das Genbearbeitungsverfahren nach Anspruch 6 oder Anspruch 7, wobei die isolierten Zellen aus Schritt (i) menschliche Stammzellen sind, vorzugsweise menschliche hämatopoetische Stammzellen.

9. Das Genbearbeitungsverfahren nach einem der Ansprüche 6 bis 8, wobei in Schritt (ii) und (iii) eine ortsspezifische Insertion einer Kassette mit 1 bis 10.000 Nukleotiden, vorzugsweise 2000 bis 10.000 Nukleotiden, in einen vorbestimmten Locus erfolgt.

10. Das Genbearbeitungsverfahren nach einem der Ansprüche 6 bis 9, wobei die Genombearbeitungseffizienz von Schritt (iv) um mindestens das Zehnfache im Vergleich zu Genombearbeitungsverfahren verbessert ist, wobei in jenen Genombearbeitungsverfahren keine Zusammensetzung nach einem der Ansprüche 1 bis 5 oder nach Anspruch 7 zu den Zellen hinzugefügt wird.

## Revendications

1. Une composition comprenant un inhibiteur de sous-unité catalytique de protéine kinase dépendant de l'ADN et un inhibiteur 53BP1, dans laquelle l'inhibiteur de sous-unité catalytique de protéine kinase dépendant de l'ADN est AZD-7648 et/ou M3814 et dans laquelle l'inhibiteur 53BP1 est présenté dans la SEQ ID NO:17.

2. La composition selon la revendication 1, dans laquelle la composition comprend de l'ADNss donneur ou de l'ADNds donneur.

3. La composition selon l'une quelconque des revendications 1 et 2, dans laquelle la composition comprend de l'ADNss donneur.

4. La composition selon l'une quelconque des revendications 1 à 3 pour une utilisation en médecine.

5. La composition selon l'une quelconque des revendications 1 à 3, dans laquelle la composition est une composition d'amélioration d'édition génétique.

6. Un procédé d'édition génétique comprenant les étapes séquentielles de :
(i) l'enrichissement de cellules isolées, suivi de l'activation desdites cellules isolées par des antigènes de surface prédéterminés ;
(ii) l'ajout d'une composition selon l'une quelconque des revendications 1 à 3 ou la revendication 5 auxdites cellules, simultanément à la transfection desdites cellules deux jours après l'étape (i) ;
(iii) l'ajout d'un stimulant auxdites cellules cinq jours après l'étape (i) ;
(iv) l'analyse de l'efficacité d'édition génomique et de la viabilité desdites cellules par tri cellulaire activé par fluorescence.

7. Le procédé d'édition génétique selon la revendication 6, dans lequel les cellules isolées de l'étape (i) sont des cellules eucaryotes.

8. Le procédé d'édition génétique selon la revendication 6 ou la revendication 7, dans lequel les cellules isolées de l'étape (i) sont des cellules souches humaines, de préférence des cellules souches hématopoïétiques humaines.

9. Le procédé d'édition génétique selon l'une quelconque des revendications 6 à 8, dans lequel, aux étapes (ii) et (iii), une insertion spécifique au site d'une cassette comprenant entre 1 et 10 000 nucléotides, de préférence entre 2000 et 10 000 nucléotides dans un locus prédéfini a lieu.

10. Le procédé d'édition génétique selon l'une quelconque des revendications 6 à 9, dans lequel l'efficacité d'édition génomique de l'étape (iv) est améliorée d'au moins 10 fois par rapport à des procédés d'édition génomique, dans lequel, dans lesdits procédés d'édition génomique, aucune composition selon l'une quelconque des revendications 1 à 5 ou selon la revendication 7 n'est ajoutée aux cellules.
